(19) Europäisches Patentamt
European Patent Office
Office européen des brevets

(11) Veröffentlichungsnummer: **0 004 315**
**B1**

(12) # EUROPÄISCHE PATENTSCHRIFT

(21) Anmeldenummer: **79100696.8**

(22) Anmeldetag: **08.03.79**

(51) Int. Cl.[3]: **C 07 D 249/08,**
C 07 D 233/54,
A 01 N 43/36

(54) **1.1-Diphenyläthenderivate, Verfahren zu ihrer Herstellung und ihre Verwendung als Mikrobizide.**

(30) Priorität: **17.03.78 CH 2931/78**

(43) Veröffentlichungstag der Anmeldung:
**03.10.79 Patentblatt 79/20**

(45) Bekanntmachung des Hinweises auf die Patenterteilung:
**25.02.81 Patentblatt 81/8**

(84) Benannte Vertragsstaaten:
**BE CH DE FR GB IT LU NL SE**

(56) Entgegenhaltungen:
**Keine**

(73) Patentinhaber: **CIBA-GEIGY AG**
**Patentabteilung Postfach**
**CH-4002 Basel (CH)**

(72) Erfinder: **Frick, Willy, Dr.**
**Alter Kirchweg 15**
**D-4148 Pfeffingen (CH)**

(72) Erfinder: **Hubele, Adolf, Dr.**
**Obere Egg 9**
**D-4465 Magden (CH)**

EP 0 004 315 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 1.1-Diphenyläthenderivate, Verfahren zu ihrer Herstellung und ihre Verwendung als Mikrobizide

Die vorliegende Erfindung betrifft 1.1Diphenyläthen-Derivate, ihre Herstellung und ihre Verwendung zur Bekämpfung von Pilzen sowie mikrobizide Mittel, die solche Verbindungen als Wirkstoffe enthalten.

Die neuen Verbindungen entsprechen der Formel I:

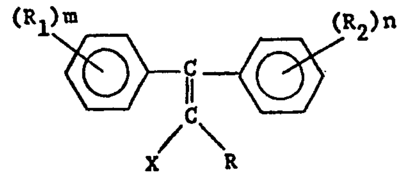

(I)

worin X Fluor oder Chlor ist, R einen gegebenenfalls durch Methyl substituierten 1,2,4-Triazolyl-1-Rest oder Imidazolyl-1-Rest bedeutet, während $R_1$ und $R_2$ unabhängig voneinander eine durch m bzw. n bestimmte Zahl gleicher oder verschiedener Substituenten aus der Gruppe Halogen, $C_1$—$C_3$ Alkyl, -$CF_3$ oder -CN darstellen, und m und n unabhängig voneinander 0,1,2 oder 3 bedeuten.

Verbindungen der Formel I lassen sich als 1.1-Diphenyl-2-haloäthene bezeichnen und stellen je nach Heterocyclus R demgemäss 1.1-Diphenyl-2-halo-2-(1,2,4-triazolyl-1)-äthene und1.1-Diphenyl-2-halo-2-(imidazolyl-1)-äthene dar.

Ihre pflanzenverträglichen Salze mit anorganischen oder organischen Säuren sowie entsprechende Metallkomplexsalze mit Cu-, Mn-, Zn-, Fe- und anderen Salzen gelten als von der Formel I mit umfasst.

Verbindungen der Formel I leiten sich in der Mehrzahl strukturell von der chemischen Gruppe der DDT-Analogen ab, besitzen jedoch keine insektizide Wirkung und werden unter natürlichen Bedingungen leicht abgebaut.

Es wurde nun überraschend gefunden, dass Verbindungen mit der Struktur der Formel I ein für die praktischen Bedürfnisse sehr günstiges Mikrobizid-Spektrum zum Schutze von Kulturpflanzen aufweisen, ohne diese durch unerwünschte Nebenwirkungen nachteilig zu beeinflussen. Kulturpflanzen seien im Rahmen vorliegender Erfindung beispielsweise Getreide (Weizen, Gerste, Roggen, Hafer, Sorghum-Hirse), Mais, Reis, Gemüse, Zuckerrüben, Soja, Erdnüsse, Obstbäume, Zierpflanzen, Reben, Hopfen, Gurkengewächse (Gurken, Kürbis, Melonen), Solanaceen wie Kartoffeln, Tabak und Tomaten, sowie auch Bananen-, Kakao- und Naturkautschuk-Gewächse.

Mit den Wirkstoffen der Formel I können an Pflanzen oder Pflanzenteilen (Früchte, Blüten, Laubwerk, Stengel, Knollen, Wurzeln) dieser und verwandter Nutzkulturen die auftretenden Pilze eingedämmt oder vernichtet werden, wobei auch später zuwachsende Pflanzenteile von derartigen Pilzen verschont bleiben. Die Wirkstoffe sind gegen die den folgenden Klassen angehörenden phytopathogenen Pilze wirksam: Phycomycetes wie Oomycetes-Arten, Basidiomycetes wie vor allem Rostpilze (z.B. Puccinia); Fungi imperfecti (z.B. Verticillium, Piricularia, Cercospora); dann aber besonders gegen die der Klasse der Ascomycetes angehörenden Erreger der echten Mehltauarten wie Erysiphe und Podosphaera sowie gegen Venturia und andere. Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Als Pflanzenfungizide bevorzugt sind solch Verbindungen der Formel I, worin X Fluor bedeutet. Diese sollen Untergruppe Ia genannt werden.

Ferner sind Verbindungen der Formel I bevorzugt, bei denen die Substituenten $R_1$ und $R_2$ eine der ortho- oder para-Stellungen der Phenylringe einnehmen. Wenn sie sich von der Untergruppe Ia ableiten, sollen solche Verbindungen Untergruppe Ib genannt werden.

Besonders bevorzugt sind solche Verbindungen der Formel I, bei denen R den unsubstituierten 1,2,4-Triazolyl-1-Rest bedeutet, vor allem solche, die sich von der Untergruppe Ib ableiten und hier Untergruppe Ic genannt werden sollen.

In der Untergruppe Ic sind solche Wirkstoffe besonders bevorzugt, bei denen mindestens in zwei der vier Positionen 2,4 und 2',4' beider Phenylringe 2 Halogenatome aus der Gruppe Fluor und Chlor stehen und in den 6 Positionen 3,5,6 und 3',5',6' Wasserstoffatome. Diese besonders bevorzugte Untergruppe soll Id genannt werden.

Sehr stark wirksame Pflanzenfungizide sind unter anderen die folgenden Einzelverbindungen:

a) $\alpha,\alpha$-Bis(4-chlorphenyl)-$\beta$-fluor-$\beta$-(1,2,4-triazolyl-1)-äthen
b) $\alpha,\alpha$-Bis(2-chlorphenyl)-$\beta$-fluor-$\beta$-(1,2,4-triazolyl-1)-äthen
c) $\alpha$-Phenyl-$\alpha$-(2,4-dichlorphenyl)-$\beta$-fluor-$\beta$-(1,2,4-triazolyl-1)-äthen
d) $\alpha$-(4-Fluorphenyl)-$\alpha$-(2,4-dichlorphenyl)-$\beta$-fluor-$\beta$-(1,2,4-triazolyl-1)-äthen
e) $\alpha,\alpha$-Bis(4-fluorphenyl)-$\beta$-fluor-$\beta$-(1,2,4-triazolyl-1)-äthen
f) $\alpha$-(4-Chlorphenyl)-$\alpha$-(2,4-dichlorphenyl)-$\beta$-fluor-$\beta$-(1,2,4-triazolyl-1)-äthen
g) $\alpha,\alpha$-Bis(2,4-dichlorphenyl)-$\beta$-fluor-$\beta$-(1,2,4-triazolyl-1)-äthen

h) $\alpha,\alpha$-Bis(2-chlor-4-fluorphenyl)-$\beta$-fluor-$\beta$-(1,2,4-triazolyl-1)-äthen.

Diese Verbindungen entfalten besonders starke Wirkung bei der Bekämpfung von echten Mehltau-Arten. Verbindungen der Formel I sind in üblichen Aufwandmengen der Fungizid-Applikation pflanzentolerant und bewirken auch keine Veränderung des Pflanzenwuchses.

Verbindungen der Formel I werden einleitend auf gleichem oder ähnlichem Wege wie DDT und seine Analogen hergestellt (vgl. z.B. DBP. 547'871).

Einer der möglichen bekannten Wege zur Herstellung von symmetrischen Diphenyläthen-Derivaten besteht in der Kondensation des entsprechend substituierten Benzolderivats mit Fluoral (oder Chloral) zu einem DDT-Analogen, aus dem mit einer Base ein Mol HX abgespalten wird unter Bildung eines DDE-Analogen II:

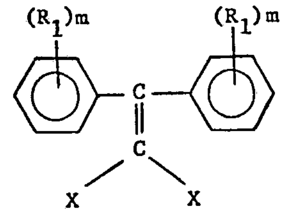

(II)

X = F oder Cl

Zur Herstellung unsymmetrischer Zwischenprodukte der Formel III

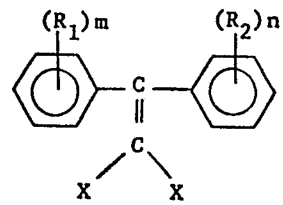

(III)

worin $R_1$ und $R_2$ bzw. m und n unterschiedliche Bedeutung haben, verfährt man analog und kondensiert molare Mengen Fluoral (bzw. Chloral) zu einem Zwischenprodukt IV, das man mit einem weiteren Benzol-Derivat kondensiert und nachfolgend ein Mol HX abspaltet:

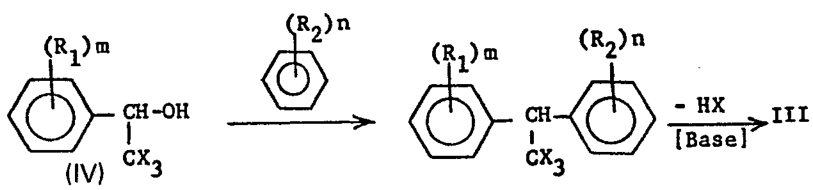


(IV)

Zur Erzielung von Verbindungen der Formel I lässt sich bei Einsatz äquimolarer Mengen eines der beiden Halogenatome an der Doppelbindung der Zwischenprodukte II bzw. III gegen den Heterocyclus R austauschen, wobei gleichzeitig 1 Mol HX abgespalten wird. Die Reaktion lässt sich häufig auch so vereinfachen, dass die HX-Abspaltung zu den Zwischenprodukten II oder III bereits in Gegenwart des Heterocyclus R durchgeführt wird, so dass II oder III in statu nascendi mit 1,2,4-Triazol bzw. Imidazol reagieren.

Zwischenprodukte der Formel IV lassen sich bekanntermassen auch durch Grignard-Reaktion eines entsprechend mit $R_1$ (bzw. $R_2$) substituierten Benzolderivats und Weiterreaktion des entstandenen Phenylmagnesiumbromid-Derivats mit wahlweise.

— Fluoral bzw. Chloral, oder

— Trifluoressigsäure bzw. Trichloressigsäure, vorzugsweise in Form ihres jeweiligen Alkalisalzes und nachfolgende katalytische Hydrierung ($H_2$/Pd) des entstandenen Trihaloacetophenons V

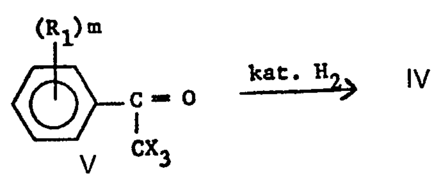


V

erhalten. Das produkt V lässt sich anstatt katalytisch auch mit $NaBH_4$ oder $LiAlH_4$ zum Carbinol IV hydrieren.

Es gibt eine Reihe weiterer Verfahren zur Herstellung der grösstenteils bekannten $\alpha,\alpha$-

Diphenyläthan- bzw. $\alpha,\alpha$-Diphenyläthen-Derivate, die in den Verfahren vorliegender Erfindung als Zwischenprodukte dienen können.

Die Erfindung betrifft ein Verfahren zur Herstellung von Verbindungen der Formel I durch Reaktion eines $\alpha,\alpha$-Diphenyl-$\beta,\beta$-dihaloäthens der Formel III mit gegebenenfalls methylsubstituiertem Imidazol bzw. 1,2,4-Triazol.

Zur Herstellung von Verbindungen der Formel I, worin X Chlor bedeutet, sind höhere Temperaturen bis zu 200°C notwendig, z.T. muss die Reaktion unter Druck erfolgen. Verbindungen, worin X Fluor bedeutet, lassen sich unter milderen Bedingungen erhalten, z.B. Raumtemperatur bis maximal 100°C.

Es werden inerte Lösungsmittel, sofern notwendig, eingesetzt. Polare Lösungsmittel wie Glyme (Aethylenglycoldimethyläther), Dimethylsulfoxid, Dimethylformamid, Dioxan, Wasser u.a. sind bevorzugt. Der Zusatz einer anorganischen Base oder eines tertiären Amins neben dem basischen Reaktanden ist empfehlenswert.

Bei unterschiedlich substituierten Phenylringen fällt ein Gemisch von cis/trans-Isomeren der Formel I an. Die reinen Antipoden besitzen verschiedenartige mikrobizide Wirkung.

In den folgenden Beispielen wird die Herstellung von Verbindungen der Formel I illustriert. Temperaturen sind durchweg in Celsius-Graden angegeben.

Beispiel 1

Herstellung von

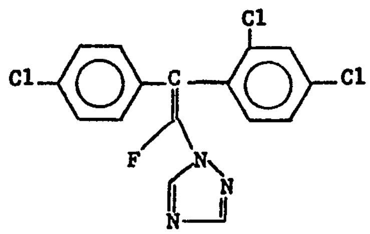

$\alpha$ - (4 - Chlorphenyl) - $\alpha$ - (2,4 - dichlorphenyl) - $\beta$ - fluor - $\beta$ - (1,2,4 - triazolyl - 1) - äthen [Verb. 16]

1 Mol (339,5 g) $\alpha$-(p-Chlorphenyl)-$\alpha$-(o,p-dichlorphenyl)-$\beta,\beta,\beta$-trifluoräthan werden in 1 Liter "Glyme" (=Aethylenglycoldimethyläther) vorgelegt und die Lösung mit 82 g Triazol (= 25% Ueberschuss) versetzt.

Bei 80—85° werden innert einer Stunde 250 ml 60%-ige KOH-Lösung zugetropft. Die Reaktionsmischung wird danach 4 Stunden bei 80° gehalten.

Nach dem Abkühlen werden die beiden Phasen getrennt, und die organische Phase wird wie üblich mit Na-Sulfat getrocknet und eingeengt. Nach dem Eindampfen hinterbleibt ein helles Oel (320 g), das am Vakuum destilliert werden kann (Sdp.: 180—185°/0.2 Torr). Es stellt ein Gemisch der zwei möglichen cis-trans-Isomeren dar. Mit Hilfe von Aether und Cyclohexan kann ein Teil des Oels zur Kristallisation gebracht werden. Das kristallisierte Produkt stellt eines der beiden Isomeren dar (Fp = 94—95°).

Beispiel 2

Herstellung von

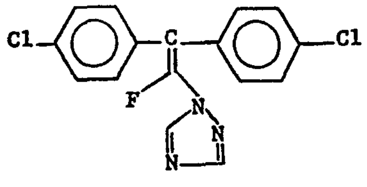

$\alpha,\alpha$ - Bis(4 - chlorphenyl) - $\beta$ - fluor - $\beta$ - (1,2,4 - triazolyl - 1) - äthen [Verb. 3]

Die Reaktion wird in analoger Weise wie im Beispiel 1 beschrieben durchgeführt.

Da bei dieser Verbindung keine geometrischen Isomeren möglich sind, fällt das Produkt nach dem Eindampfen der organischen Phase sofort kristallin aus.

Nach Umkristallisation aus Cyclohexan und Aether erhält man die analysenreine Verbindung, Fp. 105—106°.

Beispiel 3

Herstellung von

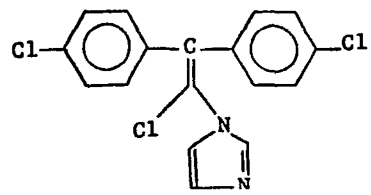

$\alpha,\alpha$ - Bis(4 - chlorphenyl) - $\beta$ - chlor - $\beta$ - (imidazolyl - 1) - äthen

1 Mol (318 g) "DDE" [=2,2-Bis(p-chlorphenyl)-1,1-dichloräthen] werden ohne Lösungsmittel mit 3 Molen Imidazol (204 g) während 20 Stunden in einem Druckrohr auf 200—210°C erhitzt.

Das Reaktionsgemisch wird dann mit ca. 1,5 Liter Wasser versetzt und mit Aether aufgenommen. Beim Eindampfen hinterlässt die Aetherlösung ein dickflüssiges Oel. Durch Hochvakuumdestillation (Sdp. 170—180°/0.005 Torr) kann dieses von dunklen und schwerflüchtigen Bestandteilen befreit werden.

Durch Umkristallisation aus Cyclohexan kann die Verbindung rein erhalten werden. Sie zeigt einen Schmelzpunkt von 104—105°C.

Beispiel 4

Herstellung von

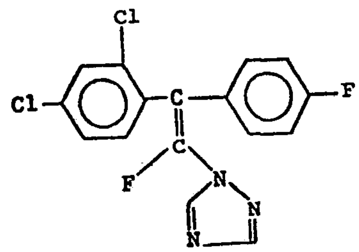

$\alpha$ - (4 - Fluorphenyl) - $\alpha$ - (2,4 - dichlorphenyl) - $\beta$ - fluor - $\beta$ - (1,2,4 - triazolyl - 1) - äthen [Verb. 23]

a) Zu 24 g Mg in 200 ml Diäthyläther werden eine Spatelspitze Jod gegeben und 175 g (1 Mol) p-Fluorchlorbenzol in 800 ml Diäthyläther derart zugetropft, dass das exotherm reagierende Gemisch am Rückfluss siedet. Nach 2 Std. wird auf 0—10°C abgekühlt und mit 152 g (1 Mol) trockenem $CF_3$—COO—K versetzt. Die erhaltene Dispersion wird über Nacht bei Raumtemperatur gerührt, gekühlt und mit 250 ml 20%ig. Chlorwasserstoffsäure versetzt. Die ätherische Phase wird abgetrennt, mit Wasser neutral gewaschen und eingeengt. Nach Vakuumdestillation des Rückstands erhält man 91 g (ca. 50%) p-Fluor-trifluoracetophenon, Sdp. 50—52°/20mbar.

b) 212 g (1 mol) p-Fluor-trifluoracetophenon werden in 1000 ml Aethanol gelöst, bei 0—10°C mit 20 g $NaBH_4$ versetzt und 2 Std. gerührt, dann weitere 4 Std. bei Raumtemperatur weitergerührt. Man fügt langsam 500 ml Wasser zu und dekantiert die überstehende klare Lösung ab, die am Rotationsverdampfer eingeengt wird. Der Rückstand wird in Diäthyläther gelöst, mit Wasser gewaschen, mit Na-Sulfat getrocknet und eingeengt. Der ölige Rückstand wird fraktioniert destilliert. Man erhält 171 g p-Fluorphenyl-trifluormethylcarbinol, Sdp. 86—90°C/20mbar.

c) 300 ml 1,3-Dichlorbenzol werden in 1000 ml konz. Schwefelsäure vorgelegt und bei 0—10°C mit 214 g (1 Mol) des unter b) erhaltenen Carbinols tropfenweise versetzt. Man rührt über Nacht bei Raumtemperatur, giesst das Reaktionsgemisch auf Eis und versetzt mit 300 ml Methylenchlorid. Die organische Phase wird mit Wasser neutral gewaschen, über Na-Sulfat getrocknet und eingeengt. Man erhält 260 g $\alpha$ - (4 - Fluorphenyl) - $\alpha$ - (2,4 - dichlorphenyl) - $\beta,\beta,\beta$ - trifluoräthan, Sdp. 95—105°C/0,13mbar.

d) 90,4 g (0,28 Mol) des unter c) erhaltenen Zwischenprodukts werden in 300 ml "Glyme" mit 21,3 g (0,31 Mole) 1,2,4-Triazol auf 80°C erhitzt (Rückfluss). Im Verlaufe 1 Std. wird eine Lösung von 47 g KOH in 100 ml Wasser zugetropft. Danach wird 6 Std. rückfliessend erhitzt. Nach dem Abkühlen wird die organische Phase mit gesättigter Kochsalz-Lösung gewaschen und eingeengt. Der viscose Rückstand wird im Hochvakuum destilliert: Sdp. 200°C/0,015mbar. Nach Umkristallisation aus Hexan erhält man das reine Endprodukt, Smp. 70—72°C.

Beispiel 5

Herstellung von

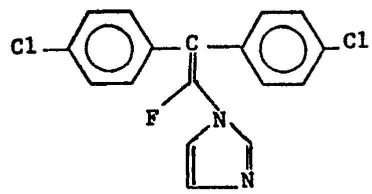

α,α - Bis(4 - chlorphenyl) - β - fluor - β (imidazolyl - 1) - äthen [Verb. 52]

1 Mol (285 g) 2,2-Bis(p-chlorphenyl)-1,1-difluor-äthylen werden in 2 Liter Dimethylformamid gelöst. In die Lösung werden bei 0—5°C 56 g fein pulverisiertes KOH eingetragen. Bis 0—5°C werden dann 67 g Imidazol, gelöst in 500 ml und Dimethylformamid innert zwei Stunden zugetropft.

Das Reaktionsgemisch wird noch 3 Stunden bei 0—5°C gehalten und dann 12 Stunden bei 20° stehengelassen. Es wird dann mit 3 Litern Wasser versetzt und mit Aether extrahiert. Nach dem Verdampfen des Aethers verbleiben 210 g des Endprodukts als fester Rückstand, der aus Aethanol umkristallisiert werden kann;
Fp. = 90—91°.

Beispiel 6

Herstellung von

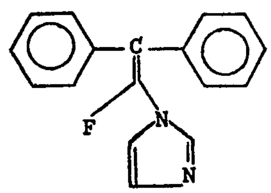

α,α - Diphenyl - β - fluor - β - (imidazolyl - 1) - äthen [Verb. 50]

1 Mol (236 g) Trifluor-diphenyl-äthan werden in 1 Liter "Diglyme" [Bis(methoxyäthyl)äther] gelöst. Zu dieser Lösung werden 75 g Imidazol, gelöst in 250 ml Wasser und 125 g KOH, gelöst in 250 ml Wasser, zugesetzt. Das Gemisch wird unter Rühren 8 Std. auf 100—110°C erhitzt.

Zur Aufarbeitung wird die Hauptmenge des Lösungsmittels im Vakuum abgedampft, der Rückstand mit 1 Liter Wasser versetzt und mit Methylenchlorid extrahiert. Die Methylenchlorid-Lösung wird mit Natriumsulfat getrocknet und eingedampft. Der Rückstand wird bei 0,5 Torr. destilliert. Dabei werden als Vorlauf ca. 30 g Ausgangsmaterial zurückgewonnen. Die Hauptmenge (ca. 200 g) destilliert zwischen 140—150°C/0,55 Torr. Beim Verrühren mit Hexan und etwas Aether fällt die Substanz kristallin an. Nach dem Trocknen am Vakuum erhält man 180 g Endprodukt, Smp. 48—50°C.

Beispiel 7

Herstellung von

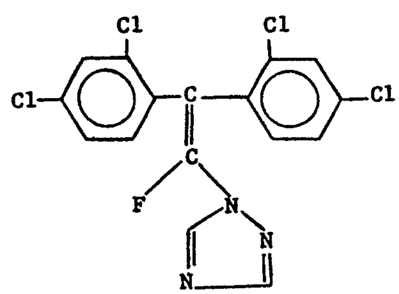

α,α - Bis - (2,4 - dichlorphenyl) - β - fluor - β - (1,2,4 - triazolyl - 1) - äthen [Verb. 4]

1 Mol (374 g) α,α-Bis-(2,4-dichlorphenyl)-β,β,β-trifluoräthan (erhalten durch Kondensation von m-Dichlorbenzol mit Fluoral in Gegenwart von konz. Schwefelsäure) werden in 800 ml "Glyme" gelöst. Unter Rühren werden 80 g 1,2,4-Triazol zugesetzt und dann bei 80—85° C 250 ml 60%-ige KOH zugetropft. Die Reaktionsmischung wird noch ca. 3 Stunden unter Rückfluss gekocht.

Nach dem Abkühlen wird die wässrige Phase abgetrennt und verworfen. Die organische Phase

wird eingedampft und der Rückstand einer Kurzwegdestillation in Hochvakuum unterworfen. Bei 180°C und 0,1 Torr destilliert die Substanz als farbloses Oel über, das beim Abkühlen zu einem festen Harz erstarrt. Die Ausbeute beträgt ca. 76%. Aus einem Gemisch von Cyclohexan und Methanol kann die Verbindung kristallisiert werden: Fp = 84—86°C.

Auf diese Art oder nach einer der oben angegebenen Methoden werden folgende Verbindungen der Formel

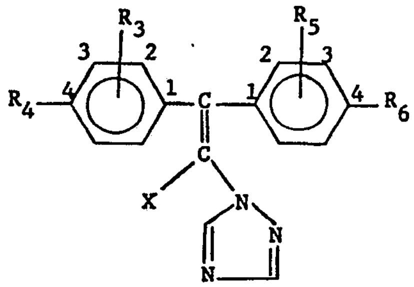

hergestellt:

| Verb. Nr. | X | $R_3$ | $R_4$ | $R_5$ | $R_6$ | Physikalische Konstante |
|---|---|---|---|---|---|---|
| 1 | F | H | H | H | H | Smp. 78—79° |
| 2 | F | 2-Cl | H | 2-Cl | H | Smp. 80—82° |
| 3 | F | H | Cl | H | Cl | Smp. 105—106° |
| 4 | F | 2-Cl | Cl | 2-Cl | Cl | Smp. 84—86° |
| 5 | F | 3-Cl | Cl | 3-Cl | Cl | |
| 6 | F | 2-$CH_3$ | Cl | 2-$CH_3$ | Cl | |
| 7 | F | H | $CH_3$ | H | $CH_3$ | Sdp. 175°/0.13mbar |
| 8 | F | 3-$CH_3$ | Cl | 3-$CH_3$ | Cl | |
| 9 | F | 3-$CF_3$ | H | 3-$CF_3$ | H | |
| 10 | F | 3-$CF_3$ | Cl | 3-$CF_3$ | Cl | |
| 11 | F | H | F | H | F | Smp. 93—94° |
| 12 | F | H | Br | H | Br | |
| 13 | F | H | CN | H | CN | viscoses Oel |
| 14 | F | 2-Cl | CN | 2-Cl | CN | |
| 15 | F | 2-$CH_3$ | CN | 2-$CH_3$ | CN | |
| 16 | F | H | Cl | 2-Cl | Cl | Smp. 94—95° |
| 17 | F | 2-Cl | H | H | Cl | Smp. 98—100° |
| 18 | F | H | Cl | 2-$CH_3$ | $CH_3$ | Sdp. 200°/0.13mbar |
| 19 | F | H | Cl | 3-$CH_3$ | $CH_3$ | Smp. 121—125° |
| 20 | F | H | Cl | H | H | Sdp. 170°/0.13mbar |
| 21 | F | H | H | 2-Cl | Cl | Smp. 88—89° |

| Verb. Nr. | X | $R_3$ | $R_4$ | $R_5$ | $R_6$ | Physikalische Konstante |
|---|---|---|---|---|---|---|
| 22 | F | H | $CH_3$ | 2-Cl | Cl | Smp. 143—144° |
| 23 | F | H | F | 2-Cl | Cl | Smp. 70—72° |
| 24 | F | H | iso $C_3H_7$ | 2-Cl | Cl | |
| 25 | F | 2-$C_2H_5$ | Cl | 2-$C_2H_5$ | Cl | |
| 26 | Cl | 2-Cl | H | 2-Cl | H | Smp. 88—91° |
| 27 | Cl | H | Cl | H | Cl | Smp. 111—113° |
| 28 | Cl | 2-Cl | Cl | 2-Cl | Cl | Sdp. 185°/0.13mbar |
| 29 | Cl | 3-Cl | Cl | 3-Cl | Cl | |
| 30 | Cl | 2-$CH_3$ | Cl | 2-$CH_3$ | Cl | |
| 31 | Cl | 3-$CH_3$ | Cl | 3-$CH_3$ | Cl | |
| 32 | Cl | 3-$CF_3$ | H | 3-$CF_3$ | H | Oel |
| 33 | Cl | 3-$CF_3$ | Cl | 3-$CF_3$ | Cl | Oel |
| 34 | Cl | H | Br | H | Br | Harz |
| 35 | Cl | H | Cl | 2-Cl | Cl | |
| 36 | Cl | 2-Cl | H | H | Cl | |
| 37 | Cl | H | H | 2-Cl | Cl | Oel |
| 38 | Cl | 2-$C_2H_5$ | Cl | 2-$C_2H_5$ | Cl | Oel |
| 39 | F | 2-Cl | F | 2-Cl | F | Smp. 82—85° |
| 40 | F | H | $CF_3$ | H | F | Oel |
| 41 | F | 2-Cl | F | H | F | Smp. 190—197°C 0.5mbar |
| 42 | F | 2-$CF_3$ | Cl | H | F | Viscos |
| 43 | F | H | Cl | H | $CF_3$ | Smp. 99—102° |
| 44 | F | 2-Cl | Cl | H | $CF_3$ | Sdp. 161—163°C/ 0.66mbar |
| 45 | F | 2-Cl | Cl | H | CN | Sdp. 157—160°/ 0.66mbar |
| 46 | F | 2-F | Cl | 2-F | Cl | Smp. 90—93° |
| 47 | F | H | Cl | H | F | Smp. 73—77° |
| 48 | F | 2-Cl | Cl | 2-Cl | F | Smp. 61—64° |
| 49 | F | 2-Cl | Cl | 2-F | F | Viscos |

Analog werden auch folgende Verbindungen der Formel

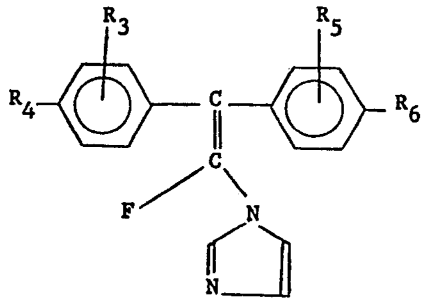

hergestellt:

| Verb. Nr. | $R_3$ | $R_4$ | $R_5$ | $R_6$ | Physikalische Konstante |
|---|---|---|---|---|---|
| 50 | H | H | H | H | Smp. 48—50° |
| 51 | 2-Cl | H | 2-Cl | H | Smp. 65—66° |
| 52 | H | Cl | H | Cl | Smp. 90—91° |
| 53 | 2-Cl | Cl | 2-Cl | Cl | Smp. 78—80° |
| 54 | 3-Cl | Cl | 3-Cl | Cl | Smp. 105—107° |
| 55 | 2-$CH_3$ | Cl | 2-$CH_3$ | Cl | |
| 56 | H | $CH_3$ | H | H | Sdp. 145—148°/ 0.13mbar |
| 57 | 3-$CH_3$ | Cl | 3-$CH_3$ | Cl | |
| 58 | 3-$CF_3$ | H | 3-$CF_3$ | H | |
| 59 | 3-$CF_3$ | Cl | 3-$CF_3$ | Cl | |
| 60 | H | F | H | F | |
| 61 | H | Br | H | Br | Smp. 98—104° |
| 62 | H | CN | H | CN | |
| 63 | 2-Cl | CN | 2-Cl | CN | Harz |
| 64 | 2-$CH_3$ | CN | 2-$CH_3$ | CN | |
| 65 | H | Cl | 2-Cl | Cl | |
| 66 | 2-Cl | H | H | Cl | Smp. 84—85° |
| 67 | H | Cl | 2-$CH_3$ | $CH_3$ | |
| 68 | H | Cl | 3-$CH_3$ | $CH_3$ | |
| 69 | H | Cl | H | H | Smp. 67—69° |
| 70 | H | H | 2-Cl | Cl | |
| 71 | H | $CH_3$ | 2-Cl | Cl | |
| 72 | 2-$CH_3$ | H | H | H | Sdp. 140—150°/ 0,25mbar |
| 73 | H | $C_2H_5$ | H | $C_2H_5$ | Oel |

Nach einer der oben angegebenen Methoden werden auch folgende mehrfach substituierte Verbindungen der Formel

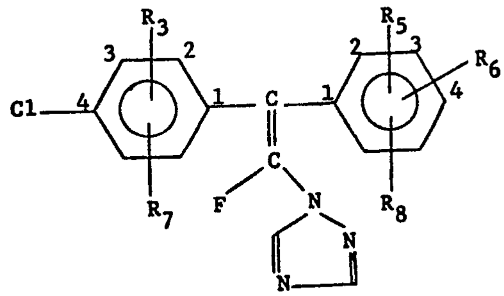

hergestellt:

| Verb. Nr. | $R_3$ | $R_7$ | $R_5$ | $R_6$ | $R_8$ | Physikalische Konstante |
|---|---|---|---|---|---|---|
| 74 | 2-Cl | 6-$CH_3$ | 2-Cl | 4-Cl | 6-$CH_3$ | Harz |
| 75 | 2-Cl | 6-Cl | 2-Cl | 4-Cl | 6-Cl | |
| 76 | 2-F | 6-F | 2-F | 4-Cl | 6-F | Oel |
| 77 | 2-$CH_3$ | 6-$CH_3$ | 2-$CH_3$ | 4-Cl | 6-$CH_3$ | Oel |
| 78 | 2-CN | 6-Cl | 2-CN | 4-Cl | 6-Cl | |
| 79 | 2-Cl | 6-$C_2H_5$ | 2-Cl | 4-Cl | 6-$C_2H_5$ | |
| 80 | 2-Cl | H | 2-$CH_3$ | 4-$CH_3$ | 6-$CH_3$ | viscos |
| 81 | H | H | 2-$CH_3$ | 4-$CH_3$ | 6-$CH_3$ | |
| 82 | H | H | 2-Cl | 4-Cl | 5-Cl | Sdp. 240°C/ 0.55mbar |

sowie die Verbindungen

Nr. 83

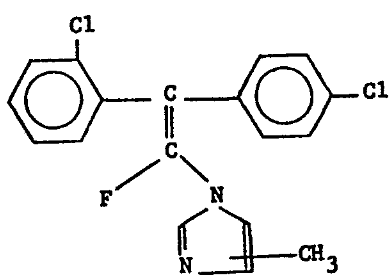

Smp 87—90°

Nr. 84

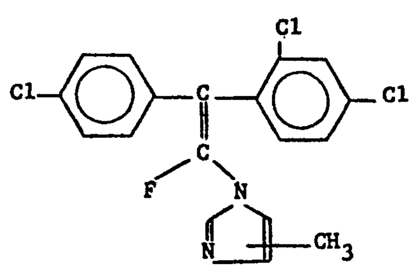

Sdp. 150—160°/0.66mbar

Die Verbindungen der Formel I können für sich allein oder zusammen mit geeigneten Trägern und/oder anderen Zuschlagstoffen verwendet werden. Geeignete Träger und Zuschlagstoffe können fest

oder flüssig sein und entsprechen den in der Formulierungstechnik üblichen Stoffen wie z.B. natürlichen oder regenerierten mineralischen Stoffen, Lösungs-, Dispergier-, Netz-, Haft-, Verdickungs-, Binde- oder Düngemitteln.

Der Gehalt an Wirkstoff in handelsfähigen Mitteln liegt zwischen 0,1 bis 90%.

Zur Applikation können die Verbindungen der Formel I in den folgenden Aufarbeitungsformen vorliegen (wobei die Gewichts-Prozentangaben in Klammern vorteilhafte Mengen an Wirkstoff darstellen):

Feste Aufarbeitungsformen: Stäubemittel und Streumittel (bis zu 10%) Granulate, Umhüllungsgranulate, Imprägnierungsgranulate und Homogengranulate, Pellets (Körner) 1 bis 80%);

Flüssige Aufarbeitungsformen:

a) in Wasser dispergierbare Wirkstoffkonzentrate: Spritzpulver (wettable powders) und Pasten (25—90% in der Handelspackung, 0,01 bis 15% in gebrauchsfertiger Lösung); Emulsions- und Lösungskonzentrate (10 bis 50%; 0.01 bis 15% in gebrauchsfertiger Lösung);
b) Lösungen (0,1 bis 20%); Aerosole

Die Wirkstoffe der Formel I vorliegender Erfindung können beispielsweise wie folgt formuliert werden:

*Stäubemittel:* Zur Herstellung eines a) 5%igen und b) 2%igen Stäubemittels werden die folgenden Stolle verwendet:

a) 5 Teile Wirkstoff
95 Teile Talkum;

b) 2 Teile Wirkstoff
1 Teil hochdisperse Kieselsäure,
97 Teile Talkum;

Die Wirkstoffe werden mit den Trägerstoffen vermischt und vermahlen und können in dieser Form zur Anwendung verstäubt werden.

*Granulat:* Zur Herstellung eines 5%igen Granulates werden die folgenden Stoffe verwendet:

5 Teile Wirkstoff
0,25 Teile Epichlorhydrin,
0,25 Teile Cetylpolyglykoläther,
3,50 Teile Polyäthylenglykol
91 Teile Kaolin (Korngrösse 0,3—0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

*Spritzpulver:* Zur Herstellung eines a) 70%igen b) 40%igen c) und d) 25%igen e) 10%igen Spritzpulvers werden folgende Bestandteile verwendet:

a) 70 Teile Wirkstoff
5 Teile Natriumdibutylnaphtylsulfonat,
3 Teile Naphthalinsulfonsäuren-Phenolsulfonsäuren-Formaldehyd-Kondensat 3:2:1,
10 Teile Kaolin,
12 Teile Champagne-Kreide;

b) 40 Teile Wirkstoff
5 Teile Ligninsulfonsäure-Natriumsalz,
1 Teil Dibutylnaphthalinsulfonsäure-Natriumsalz,
54 Teile Kieselsäure;

c) 25 Teile Wirkstoff
4,5 Teile Calcium-Ligninsulfonat,
1,9 Teile Champagne-Kreide/Hydroxyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

d) 25 Teile Wirkstoff
2,5 Teile Isooctylphenoxy-polyoxyäthylen-äthanol,
1,7 Teile Champagne-Kreide/Hydroxyäthylcellulose-Gemisch (1:1),
8,3 Teile Natriumaluminiumsilikat,
16,5 Teile Kieselgur,
46 Teile Kaolin;

e) 10 Teile Wirkstoff
3 Teile Gemisch der Natriumsalze von gesättigten Fettalkoholsulfaten,
5 Teile Naphthalinsulfonsäure/Formaldehyd-Kondensat,
82 Teile Kaolin;

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

*Emulgierbare Konzentrate:* Zur Herstellung eines 25%igen emulgierbaren Konzentrates werden folgende Stoffe verwendet:

25 Teile Wirkstoff
2,5 Teile epoxydiertes Pflanzenöl
10 Teile eines Alkylarylsulfonat/Fettalkoholpolyglykoläther-Gemisches,
5 Teile Dimethylformamid,
57,5 Teile Xylol.

Aus solchen Konzentraten können durch Verdünnen mit Wasser Emulsionen der gewünschten Konzentration hergestellt werden, die besonders zur Blattapplikation geeignet sind.

In den folgenden biologischen Beispielen sind die Wirkstoff-Konzentrationen in ppm angegeben (100 ppm = 0,01%).

Beispiel 8

Wirkung gegen Cercospora personata (=C.arachidicola) auf Erdnuss-Pflanzen

3 Wochen alte Erdnusspflanzen wurden mit einer aus Spritzpulver des Wirkstoffs hergestellten Spritzbrühe (200 ppm Aktivsubstanz) besprüht. Nach ca. 12 Stunden wurden die behandelten Pflanzen mit einer Konidiensuspension des Pilzes bestäubt. Die infizierten Pflanzen wurden dann für ca. 24 Std bei > 90% relativer Luftfeuchtigkeit inkubiert und dann im Gewächshaus bei ca.22°C aufgestellt. Der Pilzbefall wurde nach 12 Tagen ausgewertet.

Im Vergleich zur unbehandelten Kontrolle zeigten Pflanzen, die mit Wirkstoffen der Formel I behandelt waren, einen geringen oder keinen Pilzbefall. Die Verbindungen Nr. 18, 21, 23, 39, 41 verhüteten den Pilzbefall auch in einer Konzentration von 60 ppm vollständig.

Beispiel 9

Wirkung gegen Erysiphe graminis auf Gerste

Residual-protektive Wirkung

Ca. 8 cm hohe Gerstenpflanzen wurden mit einer aus Spritzpulver des Wirkstoffes hergestellten Spritzbrühe (20 ppm Aktivsubstanz) besprüht. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Die Verbindungen Formel I erzielten in der Regel starke Wirksamkeit gegen Gerstenmehltau. Mit den Verbindungen Nr. 1, 2, 3, 4, 7, 11, 16, 17, 18, 20, 21, 22, 23, 39, 41, 82 wurde der Mehltau-Befall vollständig verhindert.

Beispiel 10

Wirkung gegen Podosphaera leucotricha auf Apfeltrieben

Residual-protektive Wirkung

Apfelstecklinge mit ca. 15 cm langen Frischtrieben wurden mit einer aus Spritzpulver des Wirkstoffes hergestellten Spritzbrühe (200 ppm Aktivsubstanz) besprüht. Nach 24 Stunden wurden die behandelten Pflanzen mit einer Konidiensuspension des Pilzes infiziert und in einer Klimakammer bei 70% relativer Luftfeuchtigkeit und 20°C aufgestellt. Die Beurteilung des Pilzbefalls erfolgte 12 Tage nach

der Infektion. Mit den Wirkstoffen Nr. 2, 4, 16, 18, 21, 23, 39, 41 wurde der Pilzbefall vollständig verhütet.

Beispiel 11

Wirkung gegen Venturia inaequalis auf Apfeltrieben

Residual-protektive Wirkung

Apfelstecklinge mit 10—20 cm langen Frischtrieben wurden mit einer aus Spritzpulver des Wirkstoffes hergestellten Spritzbrühe (200 ppm Aktivsubstanz) besprüht. Nach 24 Stunden wurden die behandelten Pflanzen mit einer Konidiensuspension des Pilzes infiziert. Die Pflanzen wurden dann während 5 Tagen bei 90—100% relativer Luftfeuchtigkeit inkubiert und während 10 weiteren Tagen in einem Gewächshaus bei 20—24°C aufgestellt. Der Schorfbefall wurde 15 Tage nach der Infektion beurteilt. Mit den Wirkstoffen Nr. 21, 23, 39, 41 wurde der Pilzbefall vollständig verhütet.

Beispiel 12

Prüfung gegen samenbürtige Pilze an Getreide

Wirkung gegen helminthosporium gramineum

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testprodukte herangezogen. Mit den Verbindungen Nr. 11, 16, 21, 23 und 39 wurde das Pilzwachstum vollständig gehemmt.

Beispiel 13

Wirkung gegen Puccinia graminis f. sp. secalis auf Roggen

Residual-protektive Wirkung

Roggenpflanzen wurden 4 Tage nach der Aussaat mit einer aus Spritzpulver des Wirkstoffes hergestellten Spritzbrühe (200 ppm Aktivsubstanz) besprüht. Nach 24 Stunden wurden die behandelten Pflanzen mit einer Uredosporensuspension des Pilzes infiziert. Nach einer Inkubation während 48 Stunden bei 95—100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion. Im Vergleich mit unbehandelten, aber infizierten Kontrollpflanzen wurde mit Verbindungen der Formel I der Befall mit Rostpilz stark gehemmt oder vollständig unterbunden. Pflanzen, die mit den Wirkstoffen Nr. 11, 21, 23, 39 oder 41 behandelt worden waren, zeigten keinen Krankheitsbefall.

Auf Grund ihres breiten Pilzspektrums sind Verbindungen der einleitend genannten Untergruppe Id, wie z.B. die Wirkstoffe Nr. 21, 23 und 39, besonders hervorzuheben.

Verbindungen der Formel I lassen sich zur Verbreiterung des Wirkungsspektrums mit anderen bekannten Fungiziden, Bakteriziden, ferner auch mit Herbiziden, Insektiziden, Akariziden, Nematiziden, Pflanzenwuchsregulatoren oder Düngemitteln kombiniert anwenden.

**Patentansprüche**

1. Eine Verbindung der Formel I

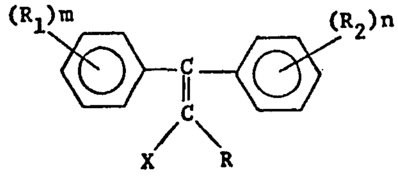

(I)

worin X Fluor oder Chlor ist, R einen gegebenenfalls durch Methyl substituierten 1,2,4-Triazolyl-1-Rest oder Imidazolyl-1-Rest bedeutet, während $R_1$ und $R_2$ unabhängig voneinander eine durch m bzw. n bestimmte Zahl gleicher oder verschiedener Substituenten aus der Gruppe Halogen, $C_1$—$C_3$ Alkyl, $-CF_3$ oder -CN darstellen, und m und n unabhängig voneinander 0,1,2 oder 3 bedeuten.

2. Eine Verbindung der Formel I gemäss Anspruch 1, worin X Fluor bedeutet.

3. Eine Verbindung der Formel I gemäss Anspruch 2, worin die Substituenten $R_1$ und $R_2$ eine der ortho- oder para-Stellungen der Phenylringe einnehmen.

4. Eine Verbindung der Formel I gemäss Anspruch 2, worin R einen 1,2,4-Triazolyl-1-Rest bedeutet.

5. Eine Verbindung der Formel I gemäss Anspruch 3, worin R einen 1,2,4-Triazolyl-1-Rest bedeutet.

6. Eine Verbindung der Formel I gemäss Anspruch 5, bei der mindestens in zwei der vier Positionen 2,4 und 2',4' beider Phenylringe 2 Halogenatome aus der Gruppe Fluor und Chlor stehen, während sich in den restlichen sechs Positionen 3,5,6 und 3',5',6' Wasserstoffatome befinden.

7. $\alpha$ - Phenyl - $\alpha$ - (2,4 - dichlorphenyl) - $\beta$ - fluor - $\beta$ - (1,2,4 - triazolyl - 1) - äthen gemäss Anspruch 6.

8. $\alpha$ - (4-Fluorphenyl) - $\alpha$ - (2,4 - dichlorphenyl) - $\beta$ - fluor - $\beta$ - (1,2,4 - triazolyl - 1) - äthen gemäss Anspruch 6.

9. $\alpha,\alpha$ - Bis(2 - chlor - 4 - fluorphenyl) - $\beta$ - fluor - $\beta$ - (1,2,4 - triazolyl - 1) - äthen gemäss Anspruch 6.

10. Verfahren zur Herstellung einer Verbindung der Formel I

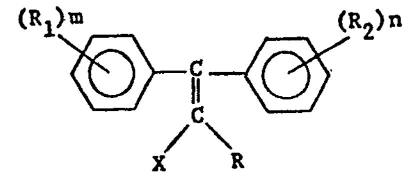

(I)

worin X Fluor oder Chlor ist, R einen gegebenenfalls durch Methyl substituierten 1,2,4-Triazolyl-1-Rest oder Imidazolyl-1-Rest bedeutet, während $R_1$ und $R_2$ unabhängig voneinander eine durch m bzw. n bestimmte Zahl gleicher oder verschiedener Substituenten aus der Gruppe Halogen, $C_1$—$C_3$ Alkyl, $-CF_3$ oder -CN darstellen, und m und n unabhängig voneinander 0,1,2 oder 3 bedeuten, durch Reaktion eines $\alpha,\alpha$-Diphenyl-$\beta,\beta$-dihaloäthens der Formel III

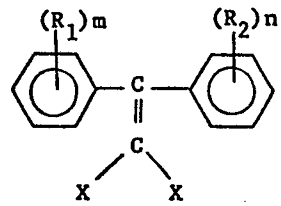

(III)

worin $R_1$, $R_2$, X, m und n die vorgenannten Bedeutungen haben, mit unsubstituiertem oder gegebenenfalls methylsubstituiertem 1,2,4-Triazol bzw. unsubstituiertem oder gegebenenfalls methylsubstituiertem Imidazol.

11. Mikrobizides Mittel enthaltend als Wirkstoff eine Verbindung der Formel I gemäss Anspruch 1, zusammen mit geeigneten Trägerstoffen und/oder oberflächenaktiven Zusätzen.

12. Mittel gemäss Anspruch 11 enthaltend eine Verbindung der Formel I gemäss Anspruch 2.

13. Mittel gemäss Anspruch 11 enthaltend eine Verbindung der Formel I gemäss Anspruch 3.

14. Mittel gemäss Anspruch 11 enthaltend eine Verbindung der Formel I gemäss Anspruch 4.

15. Mittel gemäss Anspruch 11 enthaltend eine Verbindung der Formel I gemäss Anspruch 5.

16. Mittel gemäss Anspruch 11 enthaltend eine Verbindung der Formel I gemäss Anspruch 6.

17. Mittel gemäss Anspruch 11 enthaltend die Verbindung $\alpha$ - Phenyl - $\alpha$ - (2,4 - dichlorphenyl) - $\beta$ - fluor - $\beta$ - (1,2,4 - triazolyl - 1) - äthen.

18. Mittel gemäss Anspruch 11 enthaltend die Verbindung $\alpha$ - (4 - Fluorphenyl) - $\alpha$ - (2,4 - dichlorphenyl) - $\beta$ - fluor - (1,2,4 - triazolyl - 1) - äthen.

19. Mittel gemäss Anspruch 11 enthaltend die Verbindung $\alpha,\alpha$ - Bis(2 - chlor - 4 - fluorphenyl) - $\beta$ - fluor - $\beta$ - (1,2,4 - triazolyl - 1) - äthen.

20. Verwendung einer Verbindung gemäss einem der Ansprüche 1 bis 9 zur Bekämpfung von pflanzenpathogenen Pilzen und zur Verhütung des Pilzbefalls.

**Revendications**

1. Composés de formule I:

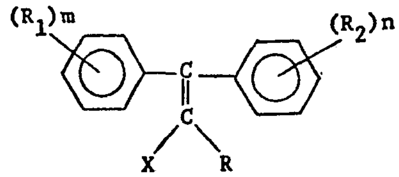

(I)

où X est un fluor ou un chlore, R représente un radical 1,2,4-triazolyl-1 ou imidazolyl-1 éventuellement substitué par un méthyle, cependant que $R_1$ et $R_2$ représentent indépendamment l'un de l'autre un nombre déterminé par m ou n de substituants semblables ou différents pris dans le groupe constitué par les halogènes, les alcoyles en $C_1$ à $C_3$, $-CF_3$ ou -CN, et m et n représentent indépendamment l'un de l'autre 0, 1, 2 ou 3.

2. Composé de formule I selon la revendication 1, où X représente un fluor.

3. Composé de formule I selon la revendication 2, où les substituants $R_1$ et $R_2$ prennent l'une des positions ortho ou para des noyaux phényle.

4. Composé de formule I selon la revendication 2, où R représente un radical 1,2,4-triazolyl-1.

5. Composé de formule I selon la revendication 3, où R représente un radical 1,2,4-triazolyl-1.

6. Composé de formule I selon la revendication 1, où se trouvent au moins dans deux des quatre positions 2,4 et 2',4' des deux noyaux phényle deux atomes d'halogène du groupe du fluor et du chlore, cependant que dans les six positions restantes 3, 5, 6 et 3', 5', 6' se trouvent les atomes d'hydrogène.

7. $\alpha$ - phényl - $\alpha$ - (2,4 - dichlorophényl) - $\beta$ - fluoro - $\beta$ - (1,2,4 - triazolyl - 1) - éthène selon la revendication 6.

8. $\alpha$ - (4 - fluorophényl) - $\alpha$ - (2,4 - dichlorophényl) - $\beta$ - fluoro - (1,2,4 - triazolyl - 1) - éthène selon la revendication 6.

9. $\alpha,\alpha$ - bis(2-chloro - 4 - fluorophényl) - $\beta$ - fluoro - $\beta$ - (1,2,4 - triazolyl - 1) - éthène selon la revendication 6.

10. Procédé de préparation d'un composé de formule I:

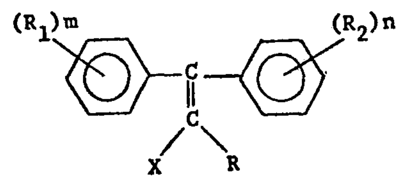

(I)

où X est un fluor ou un chlore, R représente un radical 1,2,4-triazolyl-1 ou imidazolyl-1 éventuellement substitué par un méthyle, cependant que $R_1$ et $R_2$ représentent indépendamment l'un de l'autre un nombre déterminé par m ou n de substituants semblables ou différents pris dans le groupe constitué par les halogènes, les alcoyles en $C_1$ à $C_3$, $-CF_3$ ou -CN, et m et n représentent indépendamment l'un de l'autre 0, 1, 2 ou 3, par réaction d'un $\alpha,\alpha$-diphényl-$\beta,\beta$-dihaloéthène de formule III

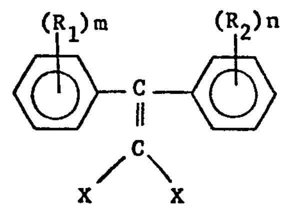

(III)

où $R_1$, $R_2$, X, m et n ont les significations mentionnées ci-dessus, avec un 1,2,4-triazole non substitué ou éventuellement substitué par un méthyle ou avec un imidazole non substitué ou éventuellement substitué par un méthyle.

11. Agent microbicide contenant comme matière active un composé de formule I selon la revendication 1, avec des supports et/ou additifs tensio-actifs appropriés.

12. Agent selon la revendication 11 contenant un composé de formule I selon la revendication 2.

13. Agent selon la revendication 11 contenant un composé de formule I selon la revendication 3.

14. Agent selon la revendication 11 contenant un composé de formule I selon la revendication 4.

15. Agent selon la revendication 11 contenant un composé de formule I selon la revendication 5.

16. Agent selon la revendication 11 contenant un composé de formule I selon la revendication 6.

17. Agent selon la revendication 11 contenant le composé $\alpha$ - phényl - $\alpha$ - (2,4 - dichlorophényl)$\beta$ - fluoro - $\beta$ - (1,2,4 - triazolyl - 1) - éthène.

18. Agent selon la revendication 11 contenant le composé α - (4 - fluorophényl) - α - (2,4 - dichlorophényl)β - fluoro - (1,2,4 - triazolyl - 1) - éthène.

19. Agent selon la revendication 11 contenant le composé α,α - bis - (2 - chloro - 4 - fluorophényl) - β - fluoro - β - (1,2,4 - triazolyl - 1) - éthène.

20. Application d'un composé selon l'une des revendications 1 à 9, à la lutte contre les champignons phytopathogènes et à l'inhibition de l'attaque des champignons.

**Claims**

1. A compound of the formula I

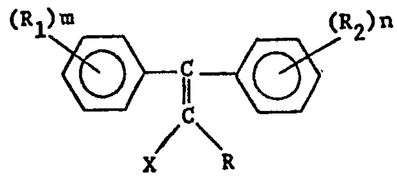

(I)

in which X is fluorine or chlorine; R is a 1,2,4-triazolyl-1 group or imidazolyl-1 group, each of which is unsubstituted or substituted by methyl; whilst $R_1$ and $R_2$ independently of one another are each a number, determined by m and n respectively, of identical or different substituents from the group: halogen, $C_1$—$C_3$ -alkyl, $CF_3$ or -CN; and m and n independently of one another are each 0, 1, 2 or 3.

2. A compound of the formula I according to Claim 1, wherein X is fluorine.

3. A compound of the formula I according to Claim 2, wherein the substituents $R_1$ and $R_2$ occupy one of the ortho- or para-positions of the phenyl rings.

4. A compound of the formula I according to Claim 2, wherein R is a 1,2,4-triazolyl-1 group.

5. A compound of the formula I according to Claim 3, wherein R is a 1,2,4-triazolyl-1 group.

6. A compound of the formula I according to Claim 5, wherein in at least two of the four positions 2, 4 and 2′, 4′ of both phenyl rings there are 2 halogen atoms from the group fluorine and chlorine, whilst in the remaining six positions 3, 5, 6 and 3′, 5′, 6′ there are hydrogen atoms.

7. α - Phenyl - α - (2,4 - dichlorophenyl) - β - fluoro - β - (1,2,4 - triazolyl - 1) - ethene according to Claim 6.

8. α - (4 - Fluorophenyl) - α - (2,4 - dichlorophenyl) - α - fluoro - (1,2,4 - triazolyl - 1) - ethene according to Claim 6.

9. α,α - Bis - (2 - chloro - 4 - fluorophenyl) - β - fluoro - β - (1,2,4- - triazolyl - 1) - ethene according to Claim 6.

10. A process for producing a compound of the formula I

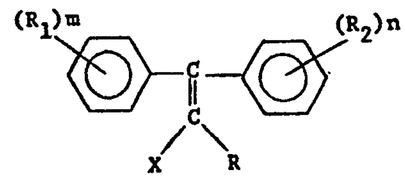

(I)

in which X is fluorine or chlorine; R is a 1,2,4-triazolyl-1 group or imidazolyl-1 group, each of which is unsubstituted or substituted by methyl; whilst $R_1$ and $R_2$ independently of one another are each a number, determined by m and n respectively, of identical or different substituents from the group: halogen, $C_1$—$C_3$-alkyl, -$CF_3$ or -CN, and m and n independently of one another are each 0, 1, 2 or 3, by reaction of an α,α-diphenyl-β,β-dihaloethene of the formula III

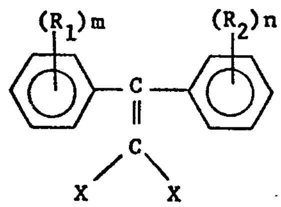

(III),

in which $R_1$, $R_2$, X, m and n are as defined in the foregoing, with unsubstituted or methyl-substituted 1,2,4-triazole or with unsubstituted or methyl-substituted imidazole.

11. A microbicidal composition containing as active substance a compound of the formula I according to Claim 1, together with suitable carriers and/or surface-active additives.

12. A composition according to Claim 11 containing a compound of the formula I according to Claim 2.

13. A composition according to Claim 11 containing a compound of the formula I according to Claim 3.

14. A composition according to Claim 11 containing a compound of the formula I according to Claim 4.

15. A composition according to Claim 11 containing a compound of the formula I according to Claim 5.

16. A composition according to Claim 11 containing a compound of the formula I according to Claim 6.

17. A composition according to Claim 11 containing the compound $\alpha$ - phenyl - $\alpha$ - (2,4 - dichlorophenyl) - $\beta$ - fluoro - $\beta$ - (1,2,4 - triazolyl - 1) - ethene.

18. A composition according to Claim 11 containing the compound $\alpha$ - (4 - fluorophenyl) - $\alpha$ - (2,4 - dichlorophenyl) - $\beta$ - fluoro - (1,2,4 - triazolyl - 1) - ethene.

19. A composition according to Claim 11 containing the compound $\alpha,\alpha$ - bis - (2 - chloro - 4 - fluorophenyl) - $\beta$ - fluoro - $\beta$ - (1,2,4 - triazolyl - 1) - ethene.

20. A method of combating phytopathogenic fungi and of preventing fungus infection which comprises applying to the locus to be protected a fungicidally effective amount of a compound according to any one of Claims 1 to 9.